(19) Europäisches Patentamt
European Patent Office
Office européen des brevets

(11) Veröffentlichungsnummer: **0 263 523 B1**

(12) **EUROPÄISCHE PATENTSCHRIFT**

(45) Veröffentlichungstag der Patentschrift: **29.12.93**

(51) Int. Cl.$^5$: **C07D 513/04**, A01N 43/90, //(C07D513/04,277:00,239:00)

(21) Anmeldenummer: **87114742.7**

(22) Anmeldetag: **09.10.87**

Die Akte enthält technische Angaben, die nach dem Eingang der Anmeldung eingereicht wurden und die nicht in dieser Patentschrift enthalten sind.

# (54) Verfahren zur Herstellung von Thiazolo-(2,3-b)-chinazolonen.

(30) Priorität: **10.10.86 DE 3634532**

(43) Veröffentlichungstag der Anmeldung: **13.04.88 Patentblatt 88/15**

(45) Bekanntmachung des Hinweises auf die Patenterteilung: **29.12.93 Patentblatt 93/52**

(84) Benannte Vertragsstaaten: **BE CH DE FR GB IT LI NL**

(56) Entgegenhaltungen:
**EP-A- 0 078 002**
**DE-A- 3 142 728**
**US-A- 4 168 380**

(73) Patentinhaber: **BASF Aktiengesellschaft**
**Carl-Bosch-Strasse 38**
**D-67063 Ludwigshafen(DE)**

(72) Erfinder: **Hamprecht, Gerhard, Dr.**
**Rote-Turm-Strasse 28**
**D-6940 Weinheim(DE)**
Erfinder: **Seybold, Günther, Dr.**
**Friedrich-Ebert-Strasse 14**
**D-6708 Neuhofen(DE)**
Erfinder: **Meyer, Norbert, Dr.**
**Dossenheimer Weg 22**
**D-6802 Ladenburg(DE)**
Erfinder: **Würzer, Bruno, Dr.**
**Rüdigerstrasse 13**
**D-6701 Otterstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Thiazolo-(2,3-b)-chinazolone der allgemeinen Formel I

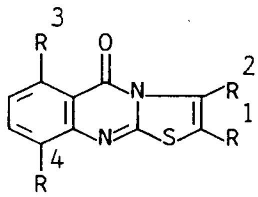

(I)

in der die Substituenten folgende Bedeutung haben.

$R^1$ Wasserstoff oder Chlor,

$R^2$ Wasserstoff, Chlor oder Methyl,

$R^3$, $R^4$ Wasserstoff, Chlor, Brom, Carboxy, $C_1$-$C_4$-Alkyl, $C_1$-$C_4$-Alkoxy, $C_1$-$C_4$-Alkylthio, gegebenenfalls durch Halogen, Methyl, Trifluormethyl, Nitro oder Methoxy substituiertes Phenoxy oder Phenylthio,

oder Salze dieser Thiazole-(2,3-b)-chinazolone, ausgenommen 6-Chlor-3-methyl-5H-thiazolo-(2,3-b)-chinazolin-5-on,9-Chlor-3-methyl-5H-thiazolo-(2,3-b)-chinazolin-5-on, 2,6-Dichlor-5H-thiazolo-(2,3-b)-chinazolin-5-on, sowie Verbindungen I, in denen beide Reste $R^3$ und $R^4$ gleichzeitig Wasserstoff bedeuten.

Ferner betrifft die Erfindung die Herstellung der neuen Verbindungen I und Mittel zur Bekämpfung unerwünschten Pflanzenwuchses und zur Beeinflussung des Pflanzenwachstums.

Aus der DE-OS 31 42 727 ist ein Verfahren zur Herstellung der Thiazolo-(2,3-b)-chinazolone I durch Umsetzung der entsprechenden Anthranilsäuren und deren Alkylestern II mit den entsprechenden Thiazolen III gemäß dem Schema

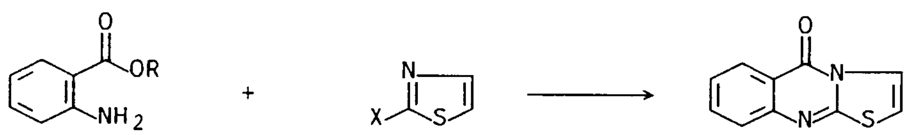

R = H, Alkyl

X = Abgangsrest z.B. Halogen

bekannt.

Dieses Verfahren ist jedoch mit befriedigendem Erfolg nicht universell anwendbar, und zwar insbesondere im Falle der Herstellung solcher Verbindungen I mit Substitution in 6- und/oder 9-Stellung.

Thiazolo-[2,3-b]-chinazolone mit Methylsubstitution in 2-Position und/oder 7- bzw. 8-Position sind aus EP-A 78 002 bekannt. Die herbizide Wirkung dieser Verbindungen ist jedoch nicht immer zufriedenstellend.

Der Erfindung lag die Aufgabe zugrunde, den geschilderten Nachteilen abzuhelfen sowie neue wirksame Herbizide bereitzustellen.

Demgemäß wurde ein Verfahren zur Herstellung der Verbindungen I gefunden, welches dadurch gekennzeichnet ist, daß man

a) entweder ein Anthranilsäureamidderivat der allgemeinen Formel II

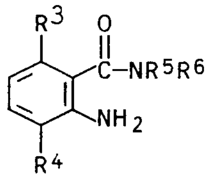

(II)

in der $R^5$ und $R^6$ für Wasserstoff oder $C_1$-$C_4$-Alkyl stehen, mit einem Thiazolderivat der allgemeinen Formel III

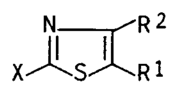

(III)

in der X Fluor, Chlor, Brom, Alkylsulfonyl oder Arylsulfonyl bedeutet, oder

b) für den Fall bestimmter Reste $R^{4'}$ aus der Gruppe $R^4$ ein Thiazolo-(2,3-b)-chinazolon der allgemeinen Formel IV

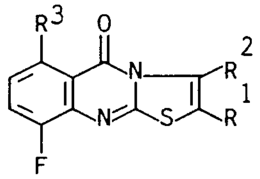

(IV)

mit einem Nucleophil $R^{4'}$-M, in dem $R^{4'}$ für Alkoxy, Alkylthio oder gegebenenfalls durch Halogen, Alkyl, Halogenalkyl, Nitro oder Alkoxy substituiertes Phenoxy oder Phenylthio steht, und M Wasserstoff, ein Alkalimetall-, Erdalkalimetallkation oder Ammoniumkation bedeutet,

umsetzt.

Außerdem wurden die neuen eingangs definierten Thiazolo-(2,3-b)chinazolone I gefunden. Weiterhin wurde gefunden, daß sich die Verbindungen I als Herbizide eignen.

Ferner wurden Verfahren und Mittel zur Bekämpfung unerwünschten Pflanzenwuchses sowie zur Beeinflussung des Pflanzenwachstums gefunden.

Die Verfahren zur Herstellung der Verbindungen I sind nach folgenden Arbeitsweisen realisierbar:

a) Umsetzung eines Anthranilsäurederivates mit einem Thiazolderivat

Die als Ausgangsstoffe eingesetzten Anthranilsäureamidderivate der Formel II und die Thiazolderivate der Formel III sind zum Teil bekannt oder können in Analogie zu bekannten Methoden hergestellt werden (J. Amer. Chem. Soc. (1952) 74, 1719; J. Sci. Ind. Research (1957) 16 B, 411 (CA. 52, 5383 d)). X in Formel III steht bevorzugt für Chlor oder Brom.

Verwendet man beispielsweise 6-Methyl-anthranilamid und 2-Chlorthiazol als Ausgangsstoffe, so kann der Reaktionsablauf durch folgende Gleichung wiedergegeben werden:

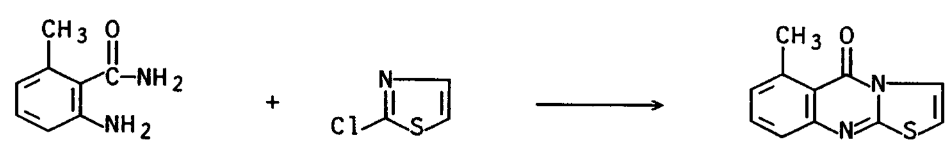

Die Umsetzung des Anthranilsäureamiderivats der Formel II mit dem Thiazolderivat der Formel III kann bei einer Temperatur im Bereich zwischen 100 und 200 °C, vorzugsweise zwischen 130 und 170 °C, durchgeführt werden. Man legt zweckmäßigerweise das Thiazol in einem inerten Lösungsmittel oder vorzugsweise in der Schmelze vor, gibt das Anthranilamid hinzu und führt dann während 30 Minuten bis 12 Stunden die Umsetzung durch. Man kann jedoch auch zuerst das Anthranilsäureamid in einem Lösungsmittel vorlegen, das Thiazol zugeben und wie beschrieben umsetzen. Die Reaktionspartner können dabei in ungefähr stöchiometrischem Verhältnis eingesetzt werden. Ein Säurezusatz ist nicht erforderlich. Als inerte Lösungsmittel kommen halogenierte Kohlenwasserstoffe wie Dichlorbenzol und Trichlorbenzol; mehrwertige Alkohole wie Glykol, Ethylglykol und Butylglykol; Ester wie Methylglykolacetat; Sulfone wie Sulfolan; Phenol und Phenolderivate wie die Kresole oder die Chlorphenole sowie Dimethylformamid in Betracht. Auch Gemische dieser Lösungsmittel können verwendet werden.

Die Umsetzung kann kontinuierlich oder diskontinuierlich zwischen 1 und 10 bar durchgeführt werden.

Zur Aufarbeitung kann das Reaktionsgemisch mit verdünnter Natriumcarbonat-, oder Ammoniaklösung gerührt und gewaschen und/oder in einem Alkohol kurz zum Sieden erhitzt und anschließend abgesaugt werden. Die so gewonnenen Endprodukte sind bereits genügend rein, um z.B. als Herbizide eingesetzt werden zu können. Man kann sie bei Bedarf natürlich weiter reinigen, z.B. durch Umkristallisation oder durch Chromatographie.

b) Umsetzung eines Fluorthiazolo(2,3-b)-chinazolons mit einem Nucleophil

Die Umsetzung, eines Fluorthiazolo(2,3-b)-chinazolons der Formel IV mit dem Nucleophil $R^{4'}$-M, in welchem $R^{4'}$ für Alkoxy, Alkylthio oder gegebenenfalls durch Halogen, Alkyl, Halogenalkyl, Nitro oder Alkoxy substituiertes Phenoxy oder Phenylthio steht und M Wasserstoff, ein Alkalimetall-, Erdalkalimetall- oder Ammoniumkation bedeutet,

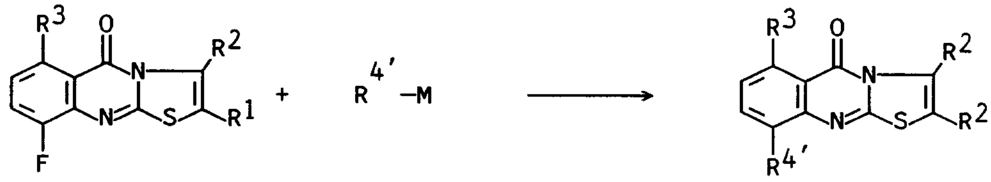

wird bei einer Temperatur im Bereich zwischen 40 und 200 °C, vorzugsweise zwischen 70 und 140 °C, durchgeführt. Bevorzugte Nuclophile sind Methanol, Ethanol, n-Propanol, Isopropanol, n-Butanol, sek.-Butanol, Isobutanol, tert.-Butanol, Methylmercaptan, Ethylmercaptan, n-Propylmercaptan, Isopropylmercaptan, n-Butylmercaptan, Isobutylmercaptan, sek.-Butylmercaptan, tert.-Butylmercaptan, Phenol, Thiophenol, 2-,3- und 4-Chlorphenol, 2-, 3- und 4-Chlorthiophenol, 2,4- und 3,4-Dlchlorphenol, 2,4- und 3,4-Dichlorphenol, 2,4- und 3,4-Dichlorthiophenol, 2-, 3- und 4-Kresol, 2-, 3- und 4-Thiokresol, 2-, 3- und 4-Nitrophenol, 2-, 3- und 4-Nitrothiophenol, 2-, 3- und 4-Methoxy-phenol, 2-, 3- und 4-Methoxy-thiophenol, 2-Chlor-4-trifluormethyl-phenol, 2-Chlor-4-trifluormethyl-thiophenol,3-Chlor-4-methoxy-phenol, 3-Chlor-4-methoxy-thiophenol, 2-Chlor-4-methoxy-phenol, 2-Chlor-4-methoxy-thiophenol und deren Alkalimetallsalze. Man legt zweckmäßigerweise das Thiazolo-(2,3-b)-chinazolon der Formel Ia in einem inerten Lösungsmittel vor, gibt dann das Nucleophil hinzu und führt dann während 30 Minuten bis 10 Stunden die Umsetzung durch. Man kann jedoch auch das Salz des Nucleophils in einem Lösungsmittel vorlegen und das Thiazolo(2,3-b)-chinazolon zugeben. Zum Binden der gegebenenfalls gebildeten Säure können die üblichen basischen Verbindungen verwendet werden. Die Reaktionspartner werden dabei ebenfalls in ungefähr stöchiometrischem Verhältnis eingesetzt. Die Umsetzung wird zweckmäßig in Gegenwart eines Lösungsmittels durchgeführt. In Betracht kommen Kohlenwasserstoffe wie Benzol, Toluol, Cyclohexan und Paraffinfraktionen wie Petrolether; Halogenkohlenwasserstoffe wie 1,1- und 1, 2-Dichlorethan, 1,1,1- und 1,1,2-Trichlorethan, Chlorbenzol, die Dichlorbenzole und die Chlortoluole; Nitroverbindungen wie Nitrobenzol und Nitroethan; Nitrile wie Acetonitril, Butyronitril und Isobutyronitril; Ether wie Dioxan; Ester wie Ethylacetat und Isobutylacetat oder Amide wie Formamid, Methylformamid und Dimethylformamid. Zweckmäßigerweise verwendet man das Lösungsmittel in einer Menge von 100 bis 2000 Gew.%, vorzugsweise 500 bis 1500 Gew.%, bezogen auf den Ausgangsstoff IV.

Die Umsetzung kann im übrigen wie unter a) beschrieben durchgeführt und aufgearbeitet werden.

Die Salze der Thiazolo(2,3-b)-chinazolone erhält man durch Protonierung mit den entsprechenden Säuren in Gegenwart eines inerten Lösungsmittels wie beispielsweise Tetrahydrofuran, Dioxan, tert.-Butyl-methyl-ether, Methylenchlorid oder Acetonitril.

Für die Salzbildung kommen anorganische Säuren, wie z.B. Chlorwasserstoffsäure, Bromwasserstoffsäure, Fluorwasserstoffsäure, Schwefelsäure, Phosphorsäure, Perchlorsäure oder organische Säuren wie beispielsweise Monochloressigsäure, Dichloressigsäure, Trichloressigsäure und Trifluoressigsäure in Betracht.

Unter den neuen Verbindungen I sind diejenigen bevorzugt, in denen

$R^3$, $R^4$ die folgende Bedeutung haben

- Wasserstoff,
- Chlor, Brom,
- Carboxy,
- Alkylreste mit 1 bis 3 Kohlenstoffatomen wie Methyl, Ethyl, n-Propyl, Isopropyl,
- Alkoxyreste mit 1 bis 3 Kohlenstoffatomen wie Methoxy, Ethoxy, n-Propoxy, Isopropoxy,
- Alkylthioreste mit 1 bis 3 Kohlenstoffatomen wie Methylthio, Ethylthio, n-Propylthio, Isopropylthio,

- einen gegebenenfalls durch Halogen, Methyl, Trifluormethyl, Nitro, Methoxy substituierten Phenoxy, 2-, 3- und 4-Chlorphenoxy, 2,4- und 3,4-Dichlorphenoxy, 2-, 3- und 4-Kresoxy, 2-Chlor-4-trifluormethylphenoxy, 2-Chlor-4-methoxy-phenoxy, 2-, 3- und 4-Nitrophenoxy, 2-, 3- und 4-Methoxy-phenoxy, 3-Chlor-4-methoxyphenoxy,
- einen Phenylthiorest wie Phenylthio, 2-, 3- und 4-Chlorphenylthio, 2,4- und 3,4-Dichlorphenylthio, 2-, 3- und 4-Methylphenylthio, 2-Chlor-4-trifluormethyl-phenylthio, 2-Chlor-4-methoxy-phenylthio, 2-, 3- und 4-Nitrophenylthio, 2-, 3- und 4-Methoxy-phenylthio, 3-Chlor-4-methoxy-phenylthio.

Besonders bevorzugte Verbindungen der Formel I sind solche, bei denen

$R^1$ Wasserstoff oder Chlor,

$R^2$ Wasserstoff, Chlor oder Methyl,

$R^3$ Wasserstoff, Chlor, Brom, Carboxy, $C_1$-$C_4$-Alkyl, $C_1$-$C_4$-Alkoxy, $C_1$-$C_4$-Alkylthio oder gegebenenfalls durch Halogen, Methyl, Trifluormethyl, Nitro oder Methoxy substituiertes Phenoxy oder Phenylthio und

$R^4$ Wasserstoff

bedeuten, ausgenommen Verbindungen I, in denen beide Reste $R^3$ und $R^4$ gleichzeitig Wasserstoff bedeuten.

Beispiele für neue Verbindungen I sind:

9-Chlor-5H-thiazolo-(2,3-b)-chinazolin-5-on
6-Methyl-5H-thiazolo-(2,3-b)-chinazolin-5-on
2-Chlor-6-methyl-5H-thiazolo-(2,3-b)-chinazolin-5-on
6-Methylmercapto-5H-thiazolo-(2,3-b)-chinazolin-5-on
6-Phenoxy-5H-thiazolo-(2,3-b)-chinazolin-5-on
6-Chlor-5H-thiazolo-(2,3-b)-chinazolin-5-on
6-Brom-5H-thiazolo-(2,3-b)-chinazolin-5-on
9-Brom-5H-thiazolo-(2,3-b)-chinazolin-5-on
6,9-Dichlor-5H-thiazolo-(2,3-b)-chinazolin-5-on
9-Methyl-5H-thiazolo-(2,3-b)-chinazolin-5-on
6-Chlor-3-methyl-5H-thiazolo-(2,3-b)-chinazolin-5-on
3,9-Dimethyl-5H-thiazolo-(2,3-b)-chinazolin-5-on
3,6-Dimethyl-5H-thiazolo-(2,3-b)-chinazolin-5-on
6-Brom-3-methyl-5H-thiazolo-(2,3-b)-chinazolin-5-on
2-Chlor-6-methoxy-5H-thiazolo-(2,3-b)-chinazolin-5-on
6-Methoxy-3-methyl-5H-thiazolo-(2,3-b)-chinazolin-5-on
2,3-Dichlor-6-methoxy-5H-thiazolo-(2,3-b)-chinazolin-5-on
2,3,6-Trichlor-5H-thiazolo-(2,3-b)-chinazolin-5-on
2,3-Dichlor-6-methyl-5H-thiazolo-(2,3-b)-chinazolin-5-on
2,3-Dichlor-9-methyl-5H-thiazolo-(2,3-b)-chinazolin-5-on
2,3,9-Trichlor-5H-thiazolo-(2,3-b)-chinazolin-5-on
2,3-Dichlor-9-methoxy-5H-thiazolo-(2,3-b)-chinazolin-5-on
2-Chlor-9-methoxy-5H-thiazolo-(2,3-b)-chinazolin-5-on
9-Methoxy-3-methyl-5H-thiazolo-(2,3-b)-chinazolin-5-on
2,3-Dichlor-9-methoxy-5H-thiazolo-(2,3-b)-chinazolin-5-on
6,9-Dimethyl-5H-thiazolo-(2,3-b)-chinazolin-5-on
6-Chlor-9-methyl-5H-thiazolo-(2,3-b)-chinazolin-5-on
9-Chlor-6-methyl-5H-thiazolo-(2,3-b)-chinazolin-5-on
6-Chlor-3,9-dimethyl-5H-thiazolo-(2,3-b)-chinazolin-5-on
6,9-Dichlor-3-methyl-5H-thiazolo-(2,3-b)-chinazolin-5-on
2,6,9-Trichlor-5H-thiazolo-(2,3-b)-chinazolin-5-on
2-Chlor-6-methylmercapto-5H-thiazolo-(2,3-b)-chinazolin-5-on
6-Ethoxy-5H-thiazolo-(2,3-b)-chinazolin-5-on
6-Ethoxy-3-methyl-5H-thiazolo-(2,3-b)-chinazolin-5-on
6-Isopropoxy-5H-thiazolo-(2,3-b)-chinazolin-5-on
9-Ethoxy-5H-thiazolo-(2,3-b)-chinazolin-5-on
9-Ethoxy-3-methyl-5H-thiazolo-(2,3-b)-chinazolin-5-on
9-Methoxy-5H-thiazolo-(2,3-b)-chinazolin-5-on
6-Methoxy-5H-thiazolo-(2,3-b)-chinazolin-5-on
6-Phenylmercapto-5H-thiazolo-(2,3-b)-chinazolin-5-on

6-(2,4-Dichlorphenoxy)-5H-thiazolo-(2,3-b)-chinazolin-5-on
6-(2-Chlor-4-trifluormethylphenoxy)-5H-thiazolo-(2,3-b)-chinazolin-5-on
6-p-Nitrophenoxy-5H-thiazolo-(2,3-b)-chinazolin-5-on
6-p-Methoxyphenoxy-5H-thiazolo-(2,3-b)-chinazolin-5-on
6-p-Methylphenylmercapto-5H-thiazolo-(2,3-b)-chinazolin-5-on
6-(2,4-Dichlorphenylmercapto)-5H-thiazolo-(2,3-b)-chinazolin-5-on.
6-Carboxy-3-methyl-5H-thiazolo-(2,3-b)-chinazolin-5-on
2,6-Dichlor-3-methyl-5H-thiazolo-(2,3-b)-chinazolin-5-on
Die folgenden Pflanzen werden auf ihr Verhalten gegenüber einigen der Verbindungen I im Gewächshaus untersucht.

| Lateinischer Name | Deutscher Name |
|---|---|
| Amaranthus retroflexus | zurückgekrümmter Fuchsschwanz |
| Avena sativa | Hafer |
| Centaurea cyanus | Kornblume |
| Chenopodium album | Weißer Gänsefuß |
| Cyperus esculentus | Erdmandel |
| Cyperus iria | - |
| Digitaria sanguinalis | Blutfingerhirse |
| Echinochloa crus-galli | Hühnerhirse |
| Euphorbia heterophylla | Wolfsmilchart |
| Galium aparine | Glettenlabkraut |
| Ipomoea spp. | Prunkwindenarten |
| Mercurialis annua | Einjähriges Bingelkraut |
| Sesbania exaltata | Turibaum |
| Setaria italica | Kolbenhirse |
| Sinapis alba | Weißer Senf |
| Solanum nigrum | Schwarzer Nachtschatten |
| Sorghum bicolor | Mohrenhirse |
| Triticum aestivum | Weizen |
| Zea mays | Mais |

Mit den neuen Verbindungen lassen sich beispielsweise folgende Pflanzen neben Kulturpflanzen bekämpfen:

| Lat. Name | Deutscher Name |
|---|---|
| Abutilon theophr. | Chinesischer Hanf |
| Amaranthus retrofl. | zurückgekr. Fuchsschwanz |
| Arachis hypogaea | Erdnuß |
| Avena sativa | Hafer |
| Centaurea cyanus | Kornblume |
| Chenopodium album | Weißer Gänsefuß |
| Cyperus esculentus | Erdmandel |
| Cyperus iria | - |
| Desmodium tortu. | - |
| Digitaria sang. | Blutfingerhirse |
| Echinochloa c.-g. | Hühnerhirse |
| Euphorbia hetero. | Wolfsmilchart |
| Galium aparine | Klettenlabkraut |
| Ipomoea spp. | Prunkwindearten |
| Lamium amplexicaule | Stengelumf. Taubnessel |
| Mercurialis annua | Einj. Bingelkraut |
| Rottboellia exalt. | - |
| Sesbania exaltata | Toribaum |
| Setaria italica | Kolbenhirse |
| Sinapis alba | Weißer Senf |
| Solanum nigrum | Schwarzer Nachtschatten |
| Sorghum bicolor | Mohrenhirse |
| Veronica spp. | Ehrenpreisarten |
| Viola tricolor | gewöhnl. Stiefmütterchen |

Zur Verbreiterung des Wirkungsspektrums und zur Erzielung synergistischer Effekte können die Thiazolo-(2,3-b)-chinazolonderivate der allgemeinen Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxischen Öle und Ölkonzentrate hergestellt werden.

Herstellungsbeispiele

Beispiel 1

9-Chlor-5H-thiazolo-(2,3-b)-chinazolin-5-on (Verbindung 1)

Eine Mischung aus 34 g 3-Chloranthranilamid und 23,9 g 2-Chlorthiazol wurde innerhalb 35 Minuten auf 170°C erhitzt und 5 Stunden nachgerührt. Nach Zugabe von weiteren 2,87 g 2-Chlorthiazol wurde noch 5 Stunden bei 170°C gerührt, und anschließend wurde bei 90°C eine Mischung von 140 ml Wasser und 60 ml 25 %iger Ammoniaklösung hinzugegeben. Das auf 25°C erkaltete Reaktionsgemisch wurde 10 Minuten gerührt und wie üblich aufgearbeitet. Es wurden 33,6 g (71 %) Verbindung 1 erhalten; Fp. 185 bis 189°C.

Beispiel 2

6-Methyl-5H-thiazolo-(2,3-b)-chinazolin-5-on (Verbindung 2)

Unter Rühren wurden 22,5 g 6-Methylanthranilamid zu 18 g 2-Chlorthiazol gegeben, die Reaktionsmischung innerhalb 20 Minuten auf 140°C erwärmt und 4 Stunden gerührt. Das Reaktionsgemisch wurde mit

Wasser unter Rühren durch Zugabe von 25 %iger Ammoniaklösung auf pH 10 eingestellt. Nach der üblichen Aufarbeitung wurden 22,3 g (69 %) Verbindung 3 erhalten; Fp. 148 bis 150 ° C.

Beispiel 3

2-Chlor-6-methyl-5H-thiazolo-(2,3-b)-chinazolin-5-on (Verbindung 3)

Unter Rühren wurden 9,75 g 6-Methylanthranilamid zu 10,8 g 2,5-Dichlorthiazol gegeben, innerhalb 30 Minuten auf 160 ° C erwärmt, 5 Stunden gerührt und wie üblich aufgearbeitet. Es wurden 10,3 g (63 %) Verbindung 4 erhalten; Fp. 162 bis 166 ° C.

Beispiel 4

6-Methylthio-5H-thiazolo-(2,3-b)-chinazolin-5-on (Verbindung 4)

7,2 g Methylmercaptan wurden bei 0 bis +10 ° C in eine Lösung von 8,4 g Kaliumhydroxid in 120 ml Methanol eingeleitet. Das Reaktionsgemisch wurde unter Erwärmen auf Raumtemperatur mit Stickstoff gespült, im Vakuum eingeengt, der Rückstand in 100 ml Dimethylformamid gelöst, mit 33 g Verbindung 2 versetzt und 3 Stunden bei 110 ° C gerührt. Nach der üblichen Aufarbeitung wurden 27 g (73 %) der Verbindung 5 erhalten; Fp. 186-189 ° C.

Beispiel 5

6-Phenoxy-5H-thiazolo-(2,3-b)-chinazolin-5-on (Verbindung 5)

11,3 g Kaliumphenolat wurden zu einer Mischung von 16,5 g der Verbindung 2 und 100 ml Dimethylformamid gegeben und 3 Stunden bei 140 ° C gerührt. Nach der üblichen Aufarbeitung wurden 15 g (68 %) Verbindung 6 erhalten, Fp. 145 bis 149 ° C.

Beispiel 6

9-Methoxy-3H-thiazolo-(2,3-b)-chinazolin-5-on (Verbindung 39)

16,6 g 3-Methoxyanthranilamid und 16,4 g 2-Bromthiazol wurden 7 Stunden bei 150 ° C gerührt. Nach der üblichen Aufarbeitung wurden 20,2 g (87 %) der Titelverbindung mit Fp. 230 bis 232 ° C erhalten.

Auf zu den Beispielen 1 bis 5 analoge Weise wurden die folgenden Verbindungen (Tabelle 1) hergestellt oder sie sind nach dieser Methode erhältlich.

Tabelle 1

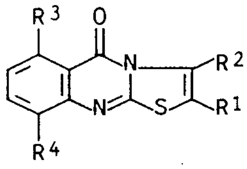

(I)

| Verbindung Nr. | $R^1$ | $R^2$ | $R^3$ | $R^4$ | Fp. [°C] |
|---|---|---|---|---|---|
| 6 | H | H | Cl | H | 213-215 |
| 7 | H | H | Br | H | 205-207 |
| 8 | H | H | H | Br | 183-184 |
| 9 | H | H | Cl | Cl | 192-196 |
| 10 | H | H | H | $CH_3$ | 180-184 |
| 11 | H | $CH_3$ | Cl | H | 274-277 |
| 12 | H | $CH_3$ | H | $CH_3$ | 188-190 |
| 13 | H | $CH_3$ | $CH_3$ | H | 217 Zers. |
| 14 | H | $CH_3$ | Br | H | 260-261 |
| 15 | Cl | H | $OCH_3$ | H | |
| 16 | H | $CH_3$ | $OCH_3$ | H | 222-224 |
| 17 | Cl | Cl | $OCH_3$ | H | |
| 18 | Cl | Cl | Cl | H | |
| 19 | Cl | Cl | $CH_3$ | H | |
| 20 | Cl | Cl | H | $CH_3$ | |
| 21 | Cl | Cl | H | Cl | |
| 22 | Cl | Cl | H | $OCH_3$ | |
| 23 | Cl | H | H | $OCH_3$ | |
| 24 | H | $CH_3$ | H | $OCH_3$ | 206-210 |
| 25 | Cl | Cl | H | $OCH_3$ | |
| 26 | H | H | $CH_3$ | $CH_3$ | |
| 27 | H | H | Cl | $CH_3$ | 173-175 |
| 28 | H | H | $CH_3$ | Cl | |
| 29 | H | $CH_3$ | Cl | $CH_3$ | |
| 30 | H | $CH_3$ | Cl | Cl | |

| Verbindung Nr. | $R^1$ | $R^2$ | $R^3$ | $R^4$ | Fp. [°C] |
|---|---|---|---|---|---|
| 31 | Cl | H | Cl | Cl | |
| 32 | H | $CH_3$ | $CH_3$ | H | |
| 33 | Cl | H | $S-CH_3$ | H | |
| 34 | H | H | O-Et | H | 145-147 |
| 35 | H | $CH_3$ | O-Et | H | 163-165 |
| 36 | H | H | $O-i-C_3H_7$ | H | 92- 96 |
| 37 | H | H | H | O-Et | |
| 38 | H | $CH_3$ | H | O-Et | |
| 39 | H | H | H | $O-CH_3$ | 230-232 |
| 40 | H | H | $O-CH_3$ | H | 162-166 |
| 41 | H | H | $S-C_6H_5$ | H | |
| 42 | H | H | 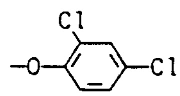 | H | |
| 43 | H | H | 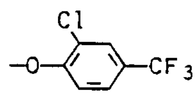 | H | |
| 44 | H | H | 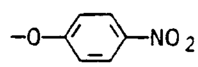 | H | |
| 45 | H | H | 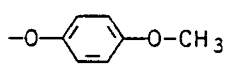 | H | |
| 46 | H | H | 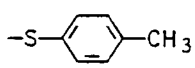 | H | |
| 47 | H | H | 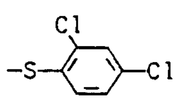 | H | |
| 48 | H | $CH_3$ | $CO_2H$ | H | 258 Zers. |
| 53 | Cl | $CH_3$ | Cl | H | |

Anwendungsbeispiele

Die herbizide Wirkung der Thiazolo-(2,3-b)-chinazolone der Formeln Ia auf das Wachstum der Testpflanzen wurde durch folgende Gewächshausversuche gezeigt.

Als Kulturgefäße dienten Plastikblumentöpfe mit 300 $cm^3$ Inhalt und lehmigem Sand mit etwa 3 % Humus als Substrat.

Die Samen der Testpflanzen wurden nach Arten getrennt flach eingesät. Unmittelbar danach erfolgte bei Vorauflaufbehandlung das Aufbringen der Wirkstoffe auf die Erdoberfläche. Die Wirkstoffe wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen und auch gleichzeitig die Wirkstoffe zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde, und verhinderte das Verdampfen leicht flüchtiger Substanzen. Die Aufwandmenge an Wirkstoff bei Vorauflaufanwendung betrug

3,0 kg/ha.

Zum Zwecke der Nachauflaufbehandlung wurden entweder direkt gesäte oder in den gleichen Gefäßen aufgewachsenen Pflanzen ausgewählt oder erst als Keimpflanzen getrennt angezogen und einige Tage vor der Behandlung in die Versuchsgefäße gepflanzt.

Je nach Wuchsform wurden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt wurden, behandelt. Die Aufwandmengen für die Nachauflaufbehandlung variierten. Sie betrugen 0,1 bis 5 kg Wirkstoff/ha, vorzugsweise 0,25 bis 2 kg Wirkstoff/ha.

Die Versuchsgefäße wurden im Gewächshaus aufgestellt, wobei für wärmeliebende Arten wärmere Bereiche (20 bis 35 °C) und für solche gemäßigter Klimate 10 bis 20 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet.

Bei Vorauflaufanwendung von 3,0 kg Wirkstoff/ha zeigte die Verbindung 10 eine gute Wirkung gegen unerwünschte Pflanzen wie Echinochloa crus-galli und Sinapis alba ohne die Kulturpflanze Hafer zu schädigen.

Ein breites Spektrum breitblättiger unerwünschter Pflanzen wie Amaranthus retroflexus, Centaurea cyanus, Echinochloa crus-galli, Galium aparine und Ipomoea spp. ließen sich mit den Verbindungen 10, 16 und 40 bei Nachauflaufapplikation sicher bekämpfen.

Gute Wirkung, gegen perennierende Cyperaceen, z.B. Cuperus esculentus, zeigten die Verbindungen 10, 40 und 24 bei Nachauflaufanwendung von 3,0 kg Wirkstoff/ha.

Schon mit niederen Aufwandmengen entfaltete Verbindung 34, im Nachauflaufverfahren angewendet, beachtliche herbizide Aktivität gegen eine Reihe unerwünschter Pflanzen wie Amaranthus retroflexus, Chenopodium album, Euphorbia heterophylla, Galium aparine, Ipomoea spp., Mercurialis annua, Sebania exaltata und Solanum bicolor.

Zur Bekämpfung von grasartigen Pflanzen wie Digitaria sanguinalis, Setaria italica und Sorghum bicolor war Verbindung 24 im Nachauflaufverfahren gut geeignet. Die Kulturpflanzen Weizen wurde dabei allenfalls unwesentlich geschädigt; die Wirkung ist selektiv.

Die Verbindungen Nr. 12 und Nr. 34 bekämpfen die Nachauflaufanwendung von 0,125 kg/ha Wirkstoff breitblättriger Unkräuter, ohne die Kulturpflanze Mais nennenswert zu schädigen.

Mit 0,25 kg/ha erzielt die Beispielsverbindung Nr. 2 die selektive Unkrautbekämpfung in Erdnüssen.

Breitblättrige und grasartige unerwünschte Pflanzen werden von Verbindung Nr. 4 bei Nachauflaufanwendung von 0,25 kg Wirkstoff/ha erfaßt, wobei die Kulturpflanzen Weizen und Mais höchstenfalls temporäre und akzeptable Schädigungen erleiden.

## Patentansprüche

**1.** Thiazolo-(2,3-b)-chinazolone der allgemeinen Formel I

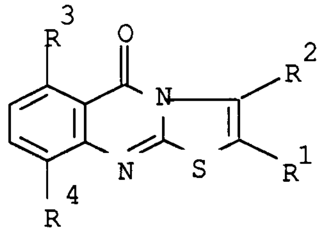

(I)

in der die Substituenten folgende Bedeutung haben:

$R^1$ Wasserstoff oder Chlor,

$R^2$ Wasserstoff, Chlor oder Methyl,

$R^3$,$R^4$ Wasserstoff, Chlor, Brom, Carboxy, $C_1$-$C_4$-Alkyl, $C_1$-$C_4$-Alkoxy, $C_1$-$C_4$-Alkylthio, gegebenenfalls durch Halogen, Methyl, Trifluormethyl, Nitro oder Methoxy substituiertes Phenoxy oder Phenylthio,

oder Salze dieser Thiazolo-(2,3-b)-chinazolone,

ausgenommen 6-Chlor-3-methyl-5H-thiazolo-(2,3-b)-chinazolin-5-on,9-Chlor-3-methyl-5H-thiazolo-(2,3-b)-chinazolin-5-on, 2,6-Dichlor-5H-thiazolo-(2,3-b)-chinazolin-5-on, sowie Verbindungen I, in denen beide Reste $R^3$ und $R^4$ gleichzeitig Wasserstoff bedeuten.

2. Thiazolo-(2,3-b)-chinazolone gemäß Anspruch 1, dadurch gekennzeichnet, daß $R^4$ Wasserstoff bedeutet.

3. Verfahren zur Herstellung von Thiazolo-(2,3-b)-chinazolonen der allgemeinen Formel I gemäß Anspruch 1, dadurch gekennzeichnet, daß man
a) entweder ein Anthranilsäureamidderivat der allgemeinen Formel II

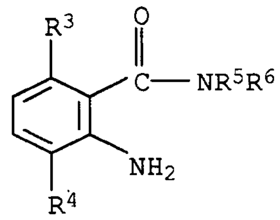

(II)

in der $R^5$ und $R^6$ für Wasserstoff oder $C_1$-$C_4$-Alkyl stehen, mit einem Thiazolderivat der allgemeinen Formel III

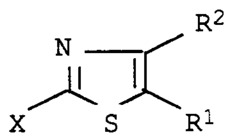

(III)

in der X Fluor, Chlor, Brom, Alkylsulfonyl oder Arylsulfonyl bedeutet, oder
b) für den Fall bestimmter Reste $R^{4'}$ aus der Gruppe $R^4$ ein Thiazolo-(2,3-b)-chinazolon der allgemeinen Formel IV

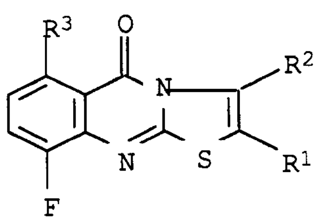

(IV)

mit einem Nucleophil $R^{4'}$-M, in welchem $R^{4'}$ für Alkoxy, Alkylthio oder gegebenenfalls durch Halogen, Alkyl, Halogenalkyl, Nitro oder Alkoxy substituiertes Phenoxy oder Phenylthio steht und M Wasserstoff, ein Alkalimetall-, Erdalkalimetallkation oder Ammoniumkation bedeutet, umsetzt.

4. Herbizid, enthaltend ein Thiazolo-(2,3-b)-chinazolon der Formel I gemäß Anspruch 1 oder deren Salze neben hierfür üblichen Trägerstoffen.

5. Verfahren zur Bekämpfung unerwünschten Pflanzenwuchses, dadurch gekennzeichnet, daß man ein Thiazolo-(2,3-b)-chinazolon der Formel I gemäß Anspruch 1 oder deren Salze auf die Pflanzen und/oder ihren Lebensraum einwirken läßt.

6. Mittel zur Beeinflussung des Pflanzenwuchses, enthaltend neben inerten Zusatzstoffen ein Thiazolo-(2,3-b)-chinazolon der Formel I gemäß Anspruch 1 oder deren Salze.

7. Verfahren zur Beeinflussung des Pflanzenwachstums, dadurch gekennzeichnet, daß man ein Thiazolo-(2,3-b)-chinazolon der Formel I gemäß Anspruch 1 oder deren Salze auf die Pflanzen und/oder ihren Lebensraum einwirken läßt.

## Claims

1. Thiazolo(2,3-b)quinazolones of the general formula I

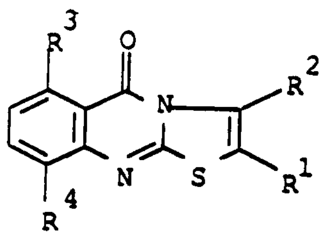

(I)

where $R^1$ is hydrogen or chlorine, $R^2$ is hydrogen, chlorine or methyl, $R^3$ and $R^4$ are each hydrogen, chlorine, bromine, carboxyl, $C_1$-$C_4$-alkyl, $C_1$-$C_4$-alkoxy, $C_1$-$C_4$-alkylthio, unsubstituted or halogen-, methyl-, trifluoromethyl-, nitro- or methoxy-substituted phenoxy or phenylthio, or salts thereof, with the exception of 6-chloro-3-methyl-5H-thiazolo(2,3-b)quinazolin-5-one, 9-chloro-3-methyl-5H-thiazolo(2,3-b)-quinazolin-5-one, 2,6-dichloro-5H-thiazolo(2,3-b)quinazoline-5-one and compounds I in which $R^3$ and $R^4$ are both hydrogen.

2. Thiazolo(2,3-b)quinazolones as claimed in claim 1, wherein $R^4$ is hydrogen.

3. A process for preparing thiazolo(2,3-b)quinazolones of the general formula I as claimed in claim 1, which comprises reacting
a) either an anthranilamide derivative of the general formula II

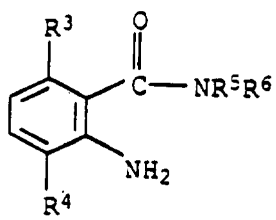

(II)

where $R^5$ and $R^6$ are each hydrogen or $C_1$-$C_4$-alkyl, with a thiazole derivative of the general formula III

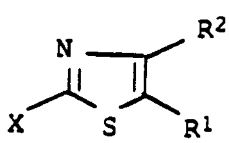

(III)

where X is fluorine, chlorine, bromine, alkylsulfonyl or arylsulfonyl or
b) for the case of certain radicals $R^{4'}$ from the group $R^4$ a thiazolo(2,3-b)quinazolone of the general formula IV

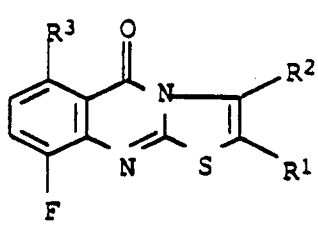

(IV)

with a nucleophile $R^{4'}$-M, where $R^{4'}$ is alkoxy, alkylthio or unsubstituted or halogen-, alkyl-, haloalkyl, nitro- or alkoxy-unsubstituted phenoxy or phenylthio and M is hydrogen or an alkali metal, alkaline earth metal or ammonium cation.

**4.** A herbicide comprising a thiazolo(2,3,-b)quinazolone of the formula I as claimed in claim 1, or a salt thereof, and conventional carriers.

**5.** A method for controlling undesirable plant growth, which comprises treating the plants and/or their habitat with thiazolo(2,3-b)quinazolone of the formula I as claimed in claim 1, or a salt thereof.

**6.** A plant growth regulator comprising, in addition to inert additives, a thiazolo(2,3-b)quinazolone of the formula I as claimed in claim 1, or a salt thereof.

**7.** A method for regulating plant growth, which comprises treating the plants and/or their habitat with a thiazolo(2,3-b)quinazolone of the formula I as claimed in claim 1, or a salt thereof,

**Revendications**

**1.** Thiazolo-(2,3-b)-quinazolones de formule générale I

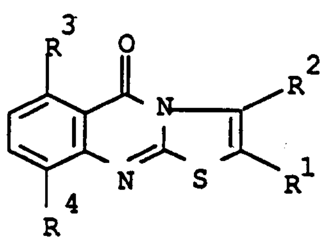

(I)

dans laquelle les substituants ont les significations suivantes :
R1, hydrogène ou chlore,
R2, hydrogène, chlore ou méthyle,
R3, R4 hydrogène, chlore, brome, carboxy, alkyle en C1-C4, alcoxy en C1-C4, alkylthio en C1-C4, phénoxy ou phénylthio, éventuellement substitué par halogène, méthyle, trifluorométhyle, nitro ou méthoxy,
ou des sels de cette thiazolo-(2,3 = b)-quinazolone à l'exeption de 6-chlore-3-méthyle-5H-thiazolo-(2,2-b)-pinazoline-5-one, 9-chlore-3-méthyle-5H-thiazolo-(2,3-b)-pinazoline-5-one, 2,6-dichlore-5H-thiazolo-(2,3-b)-pinazoline-5-one, ainsi que les composés I dans lesquels les deux restes R3 et R4 représentent simultanément l'hydrogène.

**2.** Thiazolo-(2,3-b)-quinazolones selon la revendication 1, caractérisé par le que fait que R4 représente l'hydrogène.

**3.** Procédé de préparation de thiazolo-(2,3-b)-quinazolones de formule générale I selon la revendication 1, caractérisé par le fait que l'on fait réagir :
a) soit un dérivé d'amide anthranilique de la formule générale II

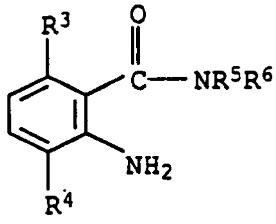

(II)

dans laquelle R5 et R6 sont mis pour hydrogène ou alkyle en C1-C4, avec un dérivé de thiazole de formule générale III

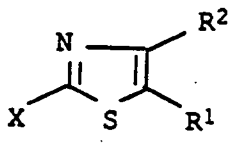

(III)

dans laquelle X représente fluor , chlore, brome, alkylsulfonyle ou arylsulfonyle, ou
b) dans le cas de reste R4' déterminé du groupe R4, une thiazolo-(2,3-b)-quinazolone de la formule générale IV

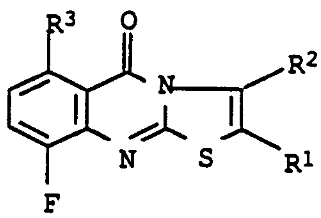

(IV)

avec un nucléophyle R4'-M, dans lequel R4' est mis pour alcoxy, alkylthio ou phénoxy ou phénylthio éventuellement substitué par halogène, alkyle, halogénalkyle, nitro ou alcoxy et M représente hydrogène, un cation métalalcalin, métal alcalino-terreux, ou un cation ammonium.

**4.** Herbicide contenant une thiazolo-(2,3-b)-quinazopone de la formule I selon la revendication 1 ou ses sels, à côté de véhicules usuels pour celà.

**5.** Procédé de lutte contre la croissance de plantes indésirables, caractérisé par le fait qu'on fait agir une thiazolo-(2,3-b)-quinazolone de formule I selon la revendication 1 ou ses sels sur les plantes et/ou leur biotope.

**6.** Moyen pour influer sur la croissance des plantes contenant, à côté d'additifs inertes, une thiazolo-(2,3-b)-quinazolone de formule I selon la revendication 1 ou ses sels.

**7.** Procédé pour influer sur la croissance des plantes, caractérisé par le fait qu'on fait agir une thiazolo-(2,3-b)-quinazolone de formule I selon la revendication 1 ou ses sels sur les plantes et/ou leur biotope.